(19) Europäisches Patentamt
European Patent Office
Office européen des brevets

(11) Numéro de publication: **0 280 583 B1**

# (12) FASCICULE DE BREVET EUROPEEN

(45) Date de publication de fascicule du brevet: **16.09.92**

(51) Int. Cl.5: **C07C 37/02**, C07C 39/15, B01J 23/72

(21) Numéro de dépôt: **88400027.4**

(22) Date de dépôt: **07.01.88**

(54) **Procédé de préparation d'hydroxybiphenyles.**

(30) Priorité: **21.01.87 FR 8700824**

(43) Date de publication de la demande:
**31.08.88 Bulletin 88/35**

(45) Mention de la délivrance du brevet:
**16.09.92 Bulletin 92/38**

(84) Etats contractants désignés:
**AT BE CH DE ES FR GB GR IT LI LU NL SE**

(56) Documents cités:
**FR-A- 2 376 836**
**US-A- 3 413 341**
**US-A- 4 490 564**

**CHEMICAL ABSTRACTS, vol. 90, no. 23, 4 juin 1979, page 629, résumé no. 186584e, Columbus, Ohio, US; & JP-A-79 22 347**

**CHEMICAL ABSTRACTS, vol. 94, no. 7, 16 février 1981, page 537, résumé no. 46883j, Columbus, Ohio, US; N.S. FIGOLI et al.: "Preparation of catalysts for phenol production"**

(73) Titulaire: **RHONE-POULENC CHIMIE**
**25, quai Paul Doumer**
**F-92408 Courbevoie Cédex(FR)**

(72) Inventeur: **Nonn, Alain**
**1, allée de la Gravière**
**F-69110 Saint Foy Les Lyon(FR)**

(74) Mandataire: **Le Pennec, Magali et al**
**RHONE-POULENC INTERSERVICES Service Brevets Chimie 25, Ouai Paul Doumer**
**F-92408 Courbevoie Cédex(FR)**

EP 0 280 583 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procedé de fabrication d'hydroxybiphényles. Elle concerne plus particulièrement un procédé de préparation de dihydroxy-4,4'biphényle.

En effet le dihydroxy-4,4'-biphényle est un intermédiaire recherché actuellement dans l'industrie des matériaux synthétiques tels que les polyesters, les polyépoxides, les polyuréthanes, comme antioxydant dans les résines ou dans l'industrie des matières colorantes.

Il est connu de préparer les hydroxybiphényles par hydrolyse des halogénébiphényles à l'aide d'une catalyse au cuivre, telle que par exemple à l'aide de sulfate de cuivre (selon le brevet US 4 475 500) ou à l'aide d'oxyde de cuivre (selon le brevet US 4 340 748) en milieu basique aqueux. Les conditions de température et la pression autogène créée par des températures aussi élevées ainsi que la faible quantité de matière première traitée à chaque opération ont incité l'homme de l'art à chercher une technique moins dangeureuse et surtout plus rentable.

Il est encore connu de nombreux procédés de préparation de composés hydroxyaryliques réalisés en phase vapeur, c'est à dire à une température supérieure à 300 °C en présence de catalyseur à base de cuivre et d'un catalyseur à base de phosphates de terres rares. Il est ainsi décrit dans le brevet US 3 752 878 un procédé de préparation de composés hydroxyaryliques dont le groupe aryle contient un ou plusieurs noyaux benzèniques à partir de composés équivalents halogénés par hydrolyse en présence d'un catalyseur à base de phosphate de lanthane ou de cerium et de cuivre qui sont précipités conjointement sous forme de polyphosphate minéral. Cette réaction est réalisée sous forme biphasique. Le catalyseur se trouvant sous forme solide et le composé halogèné à hydrolyser sous forme gazeuse car la réaction est effectuée à 500 °C. Le taux de conversion du composé de départ est faible et les conditions de réaction sont trop sévères pour être utilisables à l'échelle industrielle.

Lorsque l'on désire réaliser l'hydrolyse à une température moins élevée, telle que par exemple selon le brevet japonais 69/17372 où l'hydrolyse est réalisée à environ 200 °C, en présence de dérivés du cuivre à l'état d'oxydation deux, comme le sulfate de cuivre, le dibromure de cuivre, le diacétate de cuivre ; la durée de la réaction d'hydrolyse est particulièrement longue et atteint parfois 10 heures pour un rendement ne dépassant pas 25 %. Cette méthode n'est donc pas transférable au niveau industriel.

Le brevet US N° 4 490 564 et le résumé de la demande JA-086057 (C.A. 90 N° 23 (1979) N° 186 584 e) donnent un enseignement similaire. La demande FR-A- N° 2 376 836 décrit un procédé d'hydrolyse du chloro-2,5 bromobenzène en milieu méthanolique en présence de cuivre mais la réaction donne de mauvais résultats quand le méthanol n'est pas en large excès par rapport au dérivé bromé et le rôle du cuivre est de ralentir les réactions parasites. Enfin, le brevet US N° 3 413 341 décrit un procédé similaire qui utilise comme base des sels d'acide carboxylique faible en absence de base forte.

Ainsi depuis de nombreuses années l'industrie est à la recherche d'un procédé de préparation d'hydroxybiphényles économiquement rentable, non dangeureux à partir d'halogénobiphényles.

La présente invention a permis d'atteindre cet objectif et a pour objet un procédé industriel de préparation d'hydroxybiphényles caractérisé par le fait que l'on met en présence en phase liquide aqueuse à une température inférieure à 300 °C un bromobiphényle de formule générale (I) :

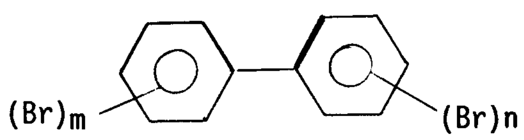

dans laquelle m et n sont des nombres entiers identiques ou différents, égal à 0,1, 2 ou 3 et dont la somme n + m est supérieure ou égale à 1 et inférieure ou égale à 4, avec une base de formule $M(OH)_p$ dans laquelle M est un métal choisi parmi les métaux alcalins ou alcalino-terreux, p est un nombre entier égal à 1 ou 2 suivant la valence du M, en présence d'un catalyseur à base de cuivre et d'un co-catalyseur choisi parmi

- les fluorures, les phosphates, les nitrates, les alcoolates, les silicates minéraux ou organiques ainsi que leurs précurseurs, les dérivés soufrés organiques, l'oxyde de carbone ou un des ses précurseurs, le méthanol, les quinoléines,
- les ammoniums quaternaires, les amines tertiaires dont la triéthylamine, les pyridines, les acides sulfoniques, les phosphines, les phosphoniums, le palladium.

Les dérivés bromés sont préparés de manière connue par tout homme de l'art par action du brome sur un biphényle à température ambiante pendant quelques heures. La variation de température, de durée, le

choix du solvant et le choix de certains catalyseurs de bromation permettent de fixer un ou plusieurs atomes de brome.

Parmi les dérivés de formule générale (I), on préfère les dérivés dans lesquels n + m est égal à 1 ou 2 et encore plus préférentiellement ceux pour lesquels n + m est égal à 2 et tout particulièrement le dibromo-4,4'biphényle.

Parmi les bases de formule $M(OH)_p$, on préfère utiliser les bases fortes alcalines et tout particulièrement la soude ou la potasse.

Parmi les catalyseurs à base de cuivre, on peut citer non limitativement le cuivre métallique, le cuivre à l'état d'oxydation un tel que l'oxyde cuivreux, les halogénures, acétate, cyanure, thiocyanate, triflate, sulfure cuivreux, ainsi que les dérivés du cuivre à l'état d'oxydation deux tels que l'oxyde, les halogénures, acétates, acétylacétonates, métaborate, isobutyrate, citrate, cyclohexylbutyrate, diméthyldithiocarbamate, hexanoate, gluconate, hydroxyde, oxalate, propionate, stéarate, sulfate, trifluoroacétylacétonate cuivriques.

On préfère cependant utiliser les oxydes ou halogénures de cuivre à l'état d'oxydation un ou deux.

Parmi les co-catalyseurs permettant de réduire soit la température, soit la durée de la réaction et objet de la protection demandée pour la présente invention, on peut choisir les fluorures les phosphates, les nitrates, les silicates, l'ensemble de ces composés sont pour leur mise en oeuvre associés à des protons ou à des cations alcalins, alcalino-terreux, métalliques par exemple de cuivre, d'argent ou organiques. Les silicates peuvent aussi être préparés in-situ dans le milieu réactionnel par action par exemple de la base forte sur de la silice ou du verre. Les phosphates peuvent également être préparés in-situ à partir de phosphates organiques.

Parmi les co-catalyseurs entièrement organiques on peut également citer par exemple les alcoolates et les alcools contenant de préférence 1 à 12 atomes de carbone, les acides carboxyliques contenant 2 à 12 atomes de carbone, les acides sulfoniques, par exemple l'acide phényl ou pyridine sulfonique, les amines tertiaires et les ammoniums, les phosphines et les phosphoniums, les quinoléines, les dérivés soufrés ainsi que l'oxyde de carbone ou un de ses précurseurs organiques tels que les formiates.

Ainsi, on peut citer pour exemplifier les co-catalyseurs de la présente invention, le diphénylsulfure, le dithiophène, le fluorure de potassium et/ou de sodium, l'acide phosphorique, l'acide nitrique, le méthylate de sodium, le formiate de méthyle, les verres, par exemple le Pyrex, la silice, l'orthoformiate de méthyle, le méthanol, l'hydroxy 8 quinoléine sulfonique 4, le bromure de tétrabutylammonium, la triéthylamine, la pyridine, l'acide pyridine sulfonique, l'acide phénylsulfonique, le chlorure de tétraphénylphosphonium, la triphénylphophine, la triphénylphosphinetrisulfonée, l'oxyde de triphénylphospine, la tributylphosphine, la tricyclohexylphosphine, le chrome héxacarbonyle, la mousse de palladium, la N-méthylpyrrolidone.

On préfère utiliser les fluorures, les phosphates, les nitrates, les formiates ou les silicates alcalins ou les formiates organiques.

Il est évident que lorque le catalyseur à base de cuivre et le co-catalyseur forment une seule entité chimique, cette dernière sera utilisée et jouera l'ensemble des deux rôles.

Parmi les conditions réactionnelles, la température est une condition essentielle du point de vue économique et elle fait l'objet du présent procédé, elle est limitée à 300 ° C et de préférence comprise entre 220 et 250 ° C. La pression utilisée est la pression autogène obtenue à la température de réaction par vaporisation des composés présents, elle peut être accrue par tout solvant inerte tel que l'azote ou tout gaz non réactif avec les composés réactionnels. La réaction ayant lieu en milieu aqueux, on utilise de préférence un mono ou polybromobiphényle à une concentration supérieure à 0,15 mole par litre d'eau et de préférence comprise entre 0,15 et 2 moles par litre d'eau. On utilise de préférence une quantité de base de formule $M(OH)_p$ libérant au moins 2 équivalents d'hydroxyle par atome de brome à hydrolyser et présentant une concentration dans l'eau comprise entre un équivalent molaire par litre et dix equivalent molaire par litre.

La quantité pondérale de catalyseur à base de cuivre est de préférence comprise entre 0,2 % et 5 % par rapport à la quantité du bromobiphényle introduite.

La quantité molaire de co-catalyseur est de préférence comprise entre 0,1 et 100 fois la quantité molaire de catalyseur. Une quantité largement excédentaire ne nuerait pas au procédé de l'invention mais cela n'apporte aucun avantage supplémentaire. La quantité utile sera simplement adaptée par l'homme de l'art à l'économie du procédé.

L'invention permet de préparer des hydroxybiphényles et parmi ceux-ci on peut citer ; l'hydroxy-4 biphényle, l'hydroxy-2 biphényle, l'hydroxy-3 biphényle, le dihydroxy-4,4'biphényle, le dihyroxy-2,2'biphényle, le dihydroxy-2,4'biphényle, le trihydroxy-2,4,4'biphényle, les tétrahydroxybiphényles.

L'invention sera plus complètement décrite à l'aide des exemples suivants qui ne doivent en aucun cas être considérés comme limitatifs de l'invention.

EXEMPLES 1 A 16 :

Mode opératoire :

On introduit dans un réacteur en Hastelloy c 276 de 75 ml les composés suivants :

- 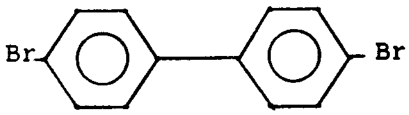 2 g (6,4 mmoles)
- $Cu_2O$ 10 mg (0,07 mmoles)
- NaOH 5N 20 ml (100 mmoles )
- Cocatalyseur 2 moles par mole de $Cu_2O$ ou 20 mg dans le cas du pyrex les gaz sont introduits par purge.

Après une purge à l'argon, fermeture de l'autoclave puis introduction éventuelle de co-catalyseur gazeux, l'autoclave est placé dans un four agité et porté à 250 ° C. Après un temps T de réaction, le réacteur est ramené à température ambiante. Le mélange réactionnel est dilué par 20 ml d'eau (rinçage du réacteur), acidifié par 25 ml d'acide sulfurique 4N et extrait à la méthylisobutylcétone (1x20 ml + 5x10 ml). La phase organique est séchée sur sulfate de sodium. Les rendements sont déterminés par chromatographie liquide haute performance.

Tableau des resultats voir page suivante :

Tableau I

| N° | Co-catalyseurs | Durée | RR (%) 4-OH | RR(%) 4,4' diOH |
|---|---|---|---|---|
| 1 | - | 3 h | 0,4 | 52,9 |
| 2 | - | 5 h | 1,5 | 85,2 |
| 3 | - | 7 h | 1,3 | 90,5 |
| 4 | Ph2S | 3 h | 0,5 | 60,6 |
| 5 | dithiophène | 3 h | 0,6 | 68,8 |
| 6 | KF | 3 h | 1,7 | 88,8 |
| 7 | NaF | 3 h | 1,4 | 93,3 |
| 8 | H3PO4 | 3 h | 2,7 | 91,2 |
| 9 | HNO3 | 3 h | 1,2 | 83,6 |
| 10 | NaCN | 3 h | 0,3 | 58,9 |
| 11 | NaOMe | 3 h | 4,2 | 83,0 |
| 12 | NaBr (1 mole par mole de Br2 (C6H4)2 | 3 h | 1,2 | 84,5 |
| 13 | HCO2Me | 3 h | 1,7 | 90,4 |
| 14 | CO à 6,4 % dans N2 1 purge | 3 h | 0,5 | 62,5 |
| 15 | Pyrex | 3 h | 1,7 | 86,9 |
| 16 | SiO2 | 3 h | 1,8 | 89,4 |

EXAMPLE 17 :

On opère comme décrit à l'exemple 1, en introduisant :

| | |
|---|---|
| - 2 g de dibromo-4,4'biphényle | (6,4 mmoles) |
| - 20 ml de NaOH 5 N | (100 mmoles) |
| - 10 mg d'oxyde cuivreux | (0,07 mmoles) |
| - 1,4 g de NaBr | (14 mmoles) |

Après 3 heures de réaction, on obtient un rendement en dihydro-4,4'biphényle de 84,7 % et un rendement en hydroxy-4 biphényle de 1,2 %.

EXAMPLE 18 :

On opère comme décrit à l'exemple 1, en hydrolysant un mélange de:

- bromo-2-biphényle
- dibromo-2,4 biphényle
- dibromo-2,4' biphényle
- dibromo-2,2' biphényle
- tribromo-2,4,4' biphényle

obtenu par bromation du bromo-2 biphényle par le brome dans le dichlorométhane en présence de 1 % de chlorure ferrique pendant 48 heures.

L'hydrolyse est conduite en présence de 0,07 mole d'oxyde cuivreux et de 2 moles de silice par mole de catalyseur, à 250 ° C dans la soude 5N.

On obtient en 3 heures un mélange de :

- hydroxy-2 biphényle
- dihydroxy-2,4 biphényle
- dihydroxy-2,4' biphényle
- dihydroxy-2,2' biphényle
- trihydroxy-2,4,4' biphényle

Les produits sont identifiés par résonance magnétique nucléaire après isolément par chromatographie phase gazeuse préparative.

EXAMPLE 19 :

Les essais suivants sont réalisés dans les mêmes conditions que les exemples 1 à 16 avec :

- 2 g de dibromo-4,4'biphényle
- 100 mg d'oxyde cuivreux
- 20 ml de NaOH 5N

à 230 ° C en utilisant le cas échéant comme co-catalyseur du butanol.

Tableau 2

| N° | Cocatalyseurs | Durée | RR (%) 4-OH | RR (%) 4,4' diOH |
|---|---|---|---|---|
| 1 | - | 7 h 30 | traces | 69,0 |
| 2 | 10 ml BuOH | 7 h 30 | 1,8 | 84,0 |

EXAMPLES 20 à 37 :

Les essais sont réalisés strictement dans les conditions de l'exemple 1 avec :

- 2 g de dibromo-4,4'-biphényle,
- 10 mg d'oxyde cuivreux,
- 20 ml de NaOH5N,
- 2 moles de cocatalyseur par mole de $Cu_2O$.

| N° | Cocatalyseur | RR(%) 4-OH | RR(%) 4,4'diOH |
|---|---|---|---|
| 20 | Sans | 0,4 | 58,1 |
| 21 | $HC(OCH_3)_3$ | 1,4 | 85,1 |
| 22 | $CH_3OH$ | 1,9 | 87,0 |
| 23 | 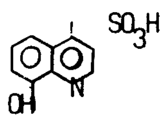 | 1,9 | 90,3 |
| 24 | $nBu_4$ NBr | 1,2 | 85,5 |
| 25 | $Et_3N$ | 0,8 | 70,0 |
| 26 | Pyridine | 2,0 | 82,8 |
| 27 | 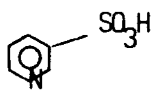 | 1,0 | 75,3 |
| 28 | 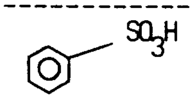 | 1,6 | 85,7 |
| 29 | $Ø_4$P Cl | 1,8 | 82,2 |
| 30 | $Ø_3$P | 1,9 | 86,3 |
| 31 | TPPTS $(HSO_3Ø)_3P$ | 1,6 | 79,0 |
| 32 | $Ø_3$P = O | 1,1 | 65,2 |
| 33 | $nBu_3P$ | 2,3 | 88,8 |
| 34 | 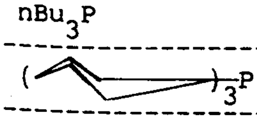 | 12,2 | 74,5 |
| 35 | $Cr(CO)_6$ | 2,3 | 85,7 |
| 36 | Pd (mousse) | 1,7 | 77,0 |
| 37 | Hydroxy-8 quinoléine | 1,4 | 90,3 |

Par ailleurs en rajoutant 0,5 ml de N-méthyl-pyrolidone à l'essai 20, on obtient, dans les mêmes conditions :

RR(4-OH) = 2,5 %

RR(4,4'diOH) = 73,9 %

EXEMPLE 38 :

Dans les mêmes conditions que l'exemple 1 on introduit

- 2 g de dibromo-4,4' biphényle,

- 19 mg de $CuF_2$ (catalyseur et cocatalyseur),
- 20 ml de NaOH5N,

après 3 h à 250 ° C on obtient un rendement en 4,4'diOH biphényle de 94,8 % et un rendement en 4-OH biphényle de 1,9 %.

## Revendications

1. Procédé de préparation d'hydroxybiphényles, caractérisé par le fait que l'on met en présence en phase liquide aqueuse à une température inférieure à 300 ° C, un bromobiphényle de formule générale (I) :

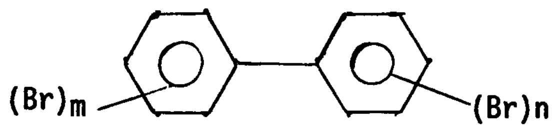

dans laquelle m et n sont des nombres entiers identiques ou différents égaux à 0,1, 2 à 3 et dont la somme n + m est supérieure ou égale à 1 et inférieure ou égale à 4, avec une base de formule $M(OH)_p$ dans laquelle M est un métal choisi parmi les métaux alcalins ou alcalino-terreux, p est un nombre entier égal à 1 ou 2 suivant la valence du métal M, en présence d'un catalyseur à base de cuivre et d'un co-catalyseur choisi parmi les fluorures, les phosphates minéraux ainsi que leurs précurseurs organiques, les nitrates, les alcoolates alcalins, alcalino-terreux, de cuivre ou d'argent, les silicates minéraux ou organiques ainsi que leurs précurseurs, les dérivés organiques soufrés, les alcools, les acides carboxyliques, l'oxyde de carbone ou un de ses précurseurs, les acides sulfoniques, les quinoléines, les ammoniums, les amines tertiaires, les phosphines, les phosphoniums, le palladium.

2. Procédé selon la revendication 1, caractérisé en ce que dans la formule (I) n + m est égal à 1 ou 2.

3. Procédé selon la revendication 1 et 2, caractérisé en ce le composé de formule (I) est le dibromo-4,4'biphényle.

4. Procédé selon la revendication 1, caractérisé en ce que le catalyseur à base de cuivre est choisi parmi les oxydes ou les halogénures du cuivre à l'état d'oxydation un ou deux.

5. Procédé selon la revendication 1, caractérisé en ce que le co-catalyseur est choisi parmi les fluorures, les phosphates alcalins, les formiates organiques et les silicates alcalins.

6. Procédé selon la revendication 5, caractérisé en ce que les silicates alcalins sont préparés in-situ à partir de silice ou de verre.

7. Procédé selon la revendication 1, caractérisé en ce que parmi les conditions réactionnelles, on préfère opérer à une température comprise entre 220 et 250 ° C.

## Claims

1. Process for the preparation of hydroxybiphenyls, characterised in that a bromobiphenyl of general formula (I):

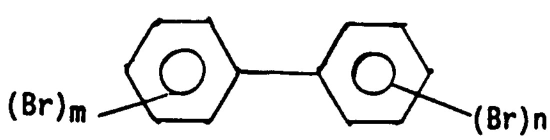

in which m and n are identical or different integers equal to 0, 1, 2 or 3 and the sum n + m of which is greater than or equal to 1 and smaller than or equal to 4, is brought into contact, in an aqueous liquid phase, at a temperature below 300 ° C, with a base of formula $M(OH)_p$ in which M is a metal chosen

from alkali metals or alkaline-earth metals, p is an integer equal to 1 or 2 depending on the valency of the metal M, in the presence of a copper-based catalyst and a cocatalyst chosen from fluorides, inorganic phosphates and their organic precursors, nitrates, alkali metal, alkaline-earth metal, copper or silver alcoholates, inorganic or organic silicates and their precursors, organic sulphur-containing derivatives, alcohols, carboxylic acids, carbon monoxide or one of its precursors, sulphonic acids, quinolines, ammoniums, tertiary amines, phosphines, phosphoniums and palladium.

**2.** Process according to claim 1, characterised in that, in formula (I), n + m is equal to 1 or 2.

**3.** Process according to claim 1 and 2, characterised in that the compound of formula (I) is 4,4'-dibromobiphenyl.

**4.** Process according to claim 1, characterised in that the copper-based catalyst is chosen from oxides or halides of copper in the oxidation state one or two.

**5.** Process according to claim 1, characterised in that the cocatalyst is chosen from fluorides, alkali metal phosphates, organic formates and alkali metal silicates.

**6.** Process according to claim 5, characterised in that the alkali metal silicates are prepared in situ starting with silica or glass.

**7.** Process according to claim 1, characterised in that among the reaction conditions, it is preferable to work at a temperature of between 220 and 250 ° C.

## Patentansprüche

**1.** Verfahren zur Herstellung von Hydroxybiphenylen, dadurch gekennzeichnet, daß man in einem wäßrigen flüssigen Medium bei einer Temperatur unter 300 ° C ein Brombiphenyl der allgemeinen Formel (I)

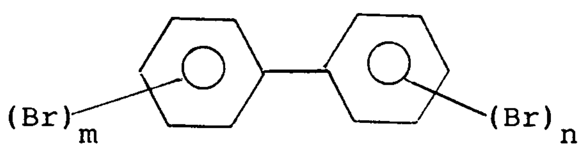

in der m und n identische oder voneinander verschiedene ganze Zahlen gleich 0, 1, 2 oder 3 sind und deren Summe n + m größer oder gleich 1 und kleiner oder gleich 4 ist, mit einer Base der Formel $M(OH)_p$, in der M ein Metall ausgewählt aus einem Alkali- oder Erdalkalimetall ist, p eine der Wertigkeit des Metalls M entsprechende ganze Zahl gleich 1 oder 2 bedeutet, in Gegenwart eines Kupferkatalysators und eines Cokatalysators ausgewählt aus den Fluoriden, mineralischen Phosphaten oder deren organischen Vorläufern, Nitraten, Alkali- oder Erdalkali-, Kupfer- oder Silberalkoholaten, anorganischen oder organischen Silikaten oder deren Vorläufern, schwefelhaltigen organischen Verbindungen, Alkoholen, carbonsäuren Kohlenmonoxid oder einem Vorläufer davon, Sulfonsäuren, Chinolinen, Ammoniumsalzen, tertiären Aminen, Phosphinen, Phosphoniumsalzen oder Palladium zur Reaktion bringt.

**2.** Verfahrem nach Anspruch 1, dadurch gekennzeichnet, daß in Formel (I) n + m gleich 1 oder 2 ist.

**3.** Verfahren nach Anspruch 1 und 2, dadurch gekennzeichnet, daß die Verbindung der Formel (I) Dibrom-4,4'-biphenyl ist.

**4.** Verfahren nach Anspruch 1, dadurch gekennzeichnet, daß der Kupferkatalysator aus einem Kupferoxid oder einem Kupferhalogenid des I- oder II-wertigen Kupfers ausgewählt ist.

**5.** Verfahren nach Anspruch 1, dadurch gekennzeichnet, daß der Cokatalysator aus einem Fluorid, Alkaliphosphat, organischen Formiat oder Alkalisilikat ausgewählt ist.

**6.** Verfahren nach Anspruch 5, dadurch gekennzeichnet, daß die Alkalisilikate in situ aus Kieselsäure oder

Glas hergestellt werden.

**7.** Verfahren nach Anspruch 1, dadurch gekennzeichnet, daß man unter den Reaktionsbedingungen vorzieht, bei einer Temperatur zwischen 220 und 250 ° C zu arbeiten.